(19) Europäisches Patentamt

European Patent Office

Office européen des brevets

(11) **EP 1 624 411 A2**

(12) **EUROPEAN PATENT APPLICATION**

(43) Date of publication:
**08.02.2006 Bulletin 2006/06**

(51) Int Cl.:
***G06T 5/40*** (2006.01)

(21) Application number: **04018747.8**

(22) Date of filing: **06.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **Gendex Corporation**
**Washington, DC 20006 (US)**

(72) Inventors:
• **Borghese, Alberto N.**
**Dipartimento di Scienza**
**Via Comelico 39**
**20135 Milano (IT)**

• **Frosio, Iuri**
**Dipartimento di Scienza dell'**
**Via Comelico 39**
**20135 Milano (IT)**

(74) Representative: **Dendorfer, Claus**
**Wächtershäuser & Hartz**
**Weinstrasse 8**
**80333 München (DE)**

(54) **Soft tissue filtering in medical images**

(57) A method is disclosed for enhancing the visibility of at least some features of a radiographic image, the features belonging to at least a first and a second category of features, the method including the steps of determining a histogram of the image, analyzing the histogram in order to determine a distinction between values of image elements that more likely show a feature of the first category and values of image elements that more likely show a feature of the second category, and applying a correction to at least some of the image elements, wherein an image element that, according to the determined distinction, more likely shows a feature of the first category is corrected differently than an image element that, according to the determined distinction, more likely shows a feature of the second category. An apparatus and a computer-readable data carrier are adapted for performing the above steps or for causing a processor to perform the above steps.

Fig. 2a

**Description**

The present invention concerns the field of radiographic imaging and in particular the field of processing a radiographic image in order to enhance the visibility of the features shown therein. In the following, the present invention will be explained using cephalic X-ray pictures as an example. Although some embodiments of the invention are specifically tailored to the processing of cephalic X-ray pictures, the present invention in general terms is not limited to such embodiments.

Cephalic radiographs are widely used by dentists, surgeons and maxillofacial radiologists for providing data on which diagnosis, surgical-planning and implant evaluation may be based. Thanks to modern digital radiographic systems, the qualitative evaluation becomes available in real-time, and the quantitative measurement and visualization of anatomical features (e.g. nasal spine, chin tip, ...), alterations in the patient's anatomy and visualization of post-operative aesthetic modifications can be automatically computed and visualized.

To take full advantage of such systems, radiograms are generally processed to obtain optimal gray level coding, using a variety of techniques, which are generally termed *image enhancement techniques.* WO 02/054349 A1 shows an example of the application of image processing techniques for enhancing digital X-ray images. A general overview of a number of known image enhancement techniques is given in the book *Digital Image Processing and Analysis* by B. Chanda and D. D. Majumder, Prentice-Hall of India Ptv. Ltd., New Delhi, 2000. When image enhancement techniques are used in the field of the present invention, it would be desirable to obtain interactive image processing rates (processing time $\leq$ 1 s) for images that are presently of the order of 4 - 5 Mpixels and may comprise even more data in the future.

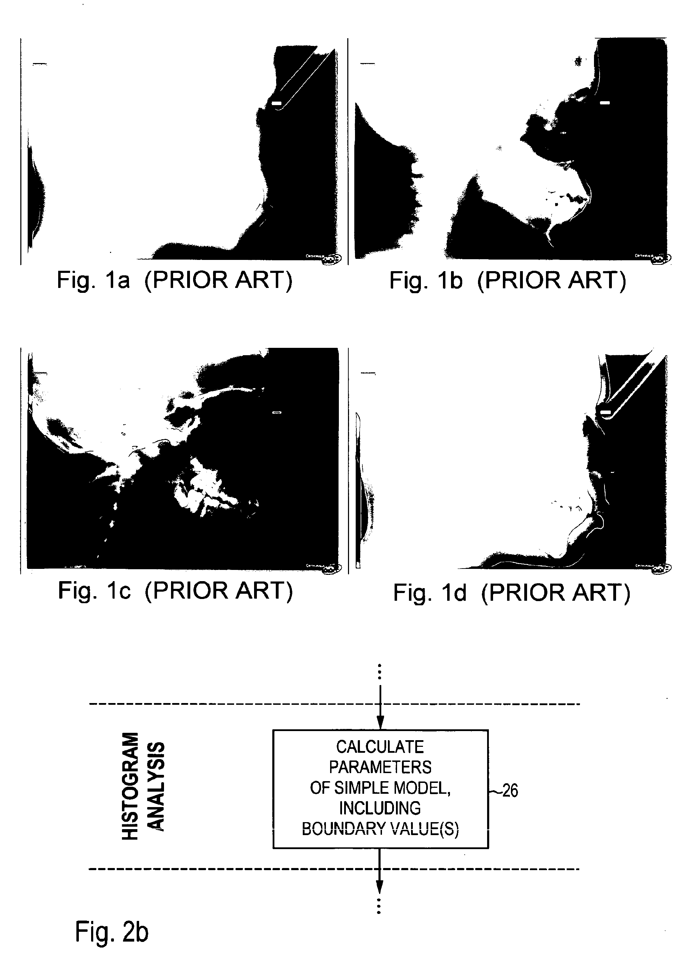

A number of image enhancement techniques have been proposed that aim to make the different features shown in the radiographic image more visible by increasing the local contrast at the border of each feature. Unsharp masking (UM) is one of the most widely used techniques, see, for example, the article "Image enhancement via adaptive unsharp masking" by Andrea Polesel, Giovanni Ramponi and V. John Mathews, *IEEE Transactions on Image Processing,* Vol. 9, No. 3, March 2000, pp. 505-510. Fig. 1d shows an example of the effect of an unsharp masking operation according to the prior art, which has been applied to the X-ray image shown in Fig. 1a.

Unsharp masking can be implemented for working in real time, but it enhances only the small features of the image and increases the noise. Moreover, it does not allow recovering underexposed images, where the dynamics of the gray levels of bone tissue is compressed: the amplitude of high frequencies in the corresponding regions is too small to become clearly visible without adding strong edge artefacts. In an overexposed image, UM identifies bone structures well, but it cannot recover the soft tissue boundary if the transition between the soft tissue and the background is smooth (large scale); this is critical for instance for the chin tip or nose profile.

Another approach to image enhancement is known as scale-space processing, see, for example, the article "Mammographic feature enhancement by multiscale feature analysis" by Adrew F. Laine, Sergio Schuler, Jian Fan and Walter Huda, *IEEE Transactions on Medical Images,* Vol. 13, No. 4, December 1994, pp. 725-740. Scale-space processing extends the capabilities of detecting features of different size but does not solve the UM problems, especially when large structures are present as in cephalic images.

Further approaches are based on morphological analysis through level-sets, morphological operators (see, e.g., the article "A multiscale morphological approach to local contrast enhancement" by Susanta Mukhopadhyay and Bhabatosh Chanda, *Signal Processing,* Vol. 80, 2000, pp. 685-696) or anisotropic filtering (see, e.g., the article "Scale-space and edge detection using anisotropic diffusion" by P. Perona and J. Malik, *IEEE Trans. PAMI,* Vol. 12, No.7, July 1990, pp. 629-639). Although these approaches provide better homogenization of the gray levels within a feature, the price to be paid is high computational complexity, which leads to processing times incompatible with real-time operation. Moreover, there is often the problem of over-enhancement or under-enhancement within different regions of an image.

Yet a further approach to image enhancement is based on re-mapping the gray levels of an image such that the image dynamic for both soft tissue and bone is maximized. A technique called gamma correction is often used in practice because gamma correction can be implemented in real-time. However, there is no single gamma value which would allow clear visibility of both kinds of soft tissue and bone. A gamma value of 0,25, which is the usual setting in many clinical applications, makes bone features clearly visible, but soft tissue darkens and tends to mix with the background; an example of this effect is shown in Fig. 1b. Gamma values larger than 1 can be used to recover overexposed soft tissue, but such a setting compresses bone dynamic.

US Patent No. 5,644,650 discloses an X-ray image displaying apparatus wherein the gradient characteristics are set to gamma > 1 in a region of interest and to gamma = 1 outside the region of interest. The width and position of the region of interest are set manually by means of setting switches. It would appear that the formulation of the gamma transformation disclosed in US Patent No. 5,644,650 is different from the formulation used in the present document.

It is also known to analyze and equalize a histogram of an image for image enhancement. Histogram equalization produces results that are generally similar to those obtained by gamma correction with a gamma value < 1 (see Fig. 1c).

More refined methods of histogram equalization, which work at a more local level, have also been proposed.

Solutions based on local statistics reframe the task as a globally constrained non-linear optimization problem, where the remapping of gray levels is constrained by levels maintaining the same ordering. Such solutions are known as local histogram equalization (see, e.g., the article "Shape preserving local histogram modification" by Vicent Caselles, Jose-Louis Lisani, Jean-Michel Morel and Guillermo Sapiro, *IEEE Transactions on Image Processing,* Vol. 8, No. 2, Feb. 1999, pp. 220-230) or homogeneity analysis (see, e.g., the article "Contrast enhancement based on a novel homogeneity measurement" by H.D. Cheng, Mei Xue and X.J. Shi, *Pattern Recognition,* Vol. 36, 2003, pp 2687-2697). These solutions, however, are often computationally intensive and may suffer from over-enhancement.

DE 199 33 776 A1 discloses an X-ray apparatus wherein the radiation of an X-ray source is controlled by means of a feedback circuit when taking an X-ray panoramic image. The intended effect is that the mean values of the successive columns of the image should be kept constant or should follow another predetermined curve. The document further discloses an embodiment in which a corresponding effect is realized by digital image processing.

The present invention has the object of providing a technique that enhances the visibility of features in a radiographic image at least for some of the features that are shown in the image. In preferred embodiments of the invention, it should be possible to implement the image enhancement method efficiently such that interactive processing times can be achieved. In further preferred embodiments, the invention should also be usable to enhance underexposed and/or overexposed images.

The invention comprises a method as defined in claim 1, an apparatus as defined in claim 22, and a computer-readable data carrier as defined in claim 23.

The dependent claims define preferred embodiments of the invention. The order in which the individual method steps are recited in the claims should not be understood as limiting the scope of the present invention. While these steps may be performed in a strictly sequential fashion in some embodiments, many other embodiments of the invention are possible in which at least some of the steps are performed in a different order or in an at least partially parallel or an at least partially interleaved (quasi-parallel) fashion.

The invention is based on the idea to enhance the visibility of at least some features of a radiographic image, the features belonging to at least a first and a second category of features, by determining a histogram of the image, analyzing the histogram in order to determine a distinction between values of image elements that more likely show a feature of the first category and values of image elements that more likely show a feature of the second category, and applying a correction to at least some of the image elements, wherein an image element that, according to the determined distinction, more likely shows a feature of the first category is corrected differently than an image element that, according to the determined distinction, more likely shows a feature of the second category.

In a particular application example, the invention may be used for processing medical X-ray images, in particular digital cephalic radiographies, so that both soft tissue features and bone features are made clearly visible under a wide range of exposures, including underexposure of bone and overexposure of soft tissue. These situations, which are frequently observed in clinical practice, lead to images in which the bone and soft tissue pixels assume similar gray levels, or in which the background tends to mix with soft tissue. In these situations, the present invention may be used to enhance the visibility of substructures inside each of these kinds of tissue of interest.

Experiments have shown a considerable enhancement of the image quality and visibility of features in typical embodiments of the present invention. Processing time was only about 1 s for 4 - 5 Mpixel images, thus making the tested embodiments well compatible with the interactive visualization rate required for clinical use. Although the default setting of certain filter parameters has turned out to be adequate for most images, real-time operation allows adjusting these parameters to recover highly underexposed or highly overexposed images or to obtain the best subjective quality. The tested embodiments also worked well with AEC (Automatic Exposure Control) to limit the X-ray dose while guaranteeing maximum image quality.

In preferred embodiments of the invention, the first category of features comprises features of soft tissue shown in the image, and the second category of features comprises features of bone shown in the image. Preferably the visibility of the features of both of these categories is enhanced, but it may also be advantageous for some applications to provide parameter settings in which only soft tissue features or only bone features are emphasized. A third category of features, that may be taken into account in some embodiments, may comprise features of the background shown in the image. It is preferred that such background features are suppressed - or at least not particularly enhanced - in some embodiments of the invention.

In some embodiments of the invention irregular image elements - e.g., image elements near a border and/or very bright and/or very dark image elements - are disregarded when calculating the histogram.

In preferred embodiments a model histogram is generated and fitted to the actual histogram of the image in a histogram analysis part of the image enhancement process. The model histogram may comprise a plurality of components, each of which preferably corresponding to one category or several categories of features shown in the image. For example, there may be a component that represents the category of soft tissue, a component that represents the category of bone, and so on.

In sophisticated embodiments, the model histogram may be formed according to a mixture model, and each of the components may be a statistical distribution. However, there are also simpler embodiments in which the model histogram is composed of a number of segments, each segment corresponding to one component. Each segment may, for example, be a straight line segment or a part of a parabola. It is preferred that each such segment is defined by a simple mathematical equation, e.g., a linear or quadratic or cubic equation.

The process of fitting the model histogram to the actual histogram preferably maximizes the likelihood of the observed data. An iterative approximation process may be used in some embodiments.

In preferred embodiments of the invention, histogram analysis produces at least one boundary value that marks the boundary between two categories of features. The correction applied to each image element is preferably influenced by the distinction of whether the value of this image element is below or above the boundary value. In particular, the correction may comprise a gamma correction with at least two different gamma correction values. In addition, the correction may comprise a linear stretching and/or a correction of saturation.

The gamma correction values that are used in the image correction step may, in some embodiments, be smoothed spatially in order to avoid artifacts in the corrected image. A two-dimensional look-up table may be used to speed up the correction process.

It is preferred that each image element that is processed according to the present invention is an individual pixel. However, there are also embodiments of the invention in which the image elements represent clusters of pixels or have been generated from the original image in a pre-processing step. When the image elements correspond to individual pixels, then the value of each image element is preferably the gray level of the corresponding pixel. In embodiments that include a pre-processing of the original image, different notions of the value of an image element (e.g., the mean gray level of a cluster of pixels or a value that emphasizes certain kinds of information contained in the image) may be used.

The apparatus of the present invention may, for example, be a digital X-ray apparatus or an image processing apparatus or any other data processing apparatus, including a suitably programmed personal computer or workstation. The computer-readable data carrier may be, without limitation, a material data carrier like, e.g., a hard disk or a CD-ROM or a semiconductor memory, or an immaterial data carrier like, e.g., a carrier wave or a signal transmitted through a computer network.

In preferred embodiments, the apparatus and the data carrier comprise features that correspond to the features set forth in the present description and/or in the dependent method claims.

Further features, advantages and objects of the present invention will be apparent from the following detailed description of a number of sample embodiments. Reference is made to the attached drawings. The drawings show:

Fig. 1a a digital radiographic image,

Fig. 1b - Fig. 1d the image of Fig. 1a after processing according to three different image enhancement techniques of the prior art,

Fig. 2a a flow diagram of an embodiment of the method of the present invention,

Fig. 2b a flow diagram of an alternative embodiment of the histogram analysis part of the method of Fig. 2a,

Fig. 3a a typical histogram of a lateral, cephalic radiography with six marked peaks,

Fig. 3b a working histogram plotted for eighteen different cephalic lateral radiographies,

Fig. 4a - Fig. 4h diagrams illustrating an iterative process of fitting a model histogram to a given histogram and determining a respective boundary value,

Fig. 5 a diagram as in Fig. 4a for an alternative embodiment in which a simplified model histogram is used,

Fig. 6a a digital radiographic image,

Fig. 6b - Fig. 6d examples of gamma correction maps that have been generated from the image of Fig. 6a,

Fig. 6e the image of Fig. 6a after gamma correction according to the gamma correction map of Fig. 6b,

Fig. 6f the image of Fig. 6a after gamma correction according to the gamma correction map of Fig. 6d,

Fig. 7a - Fig. 7j pairs of digital radiographic images, each image being shown in an original version (Fig. 7a, Fig.

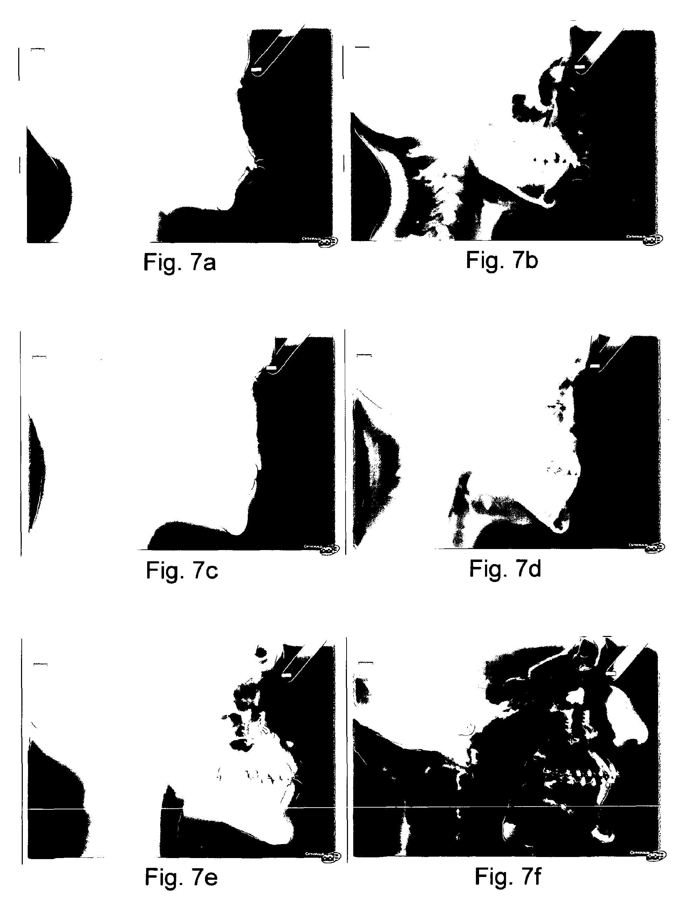

7c, Fig. 7e, Fig. 7g, Fig. 7i) and after image enhancement according to an embodiment of the present invention (Fig. 7b, Fig. 7d, Fig. 7f, Fig. 7h, Fig. 7j), and

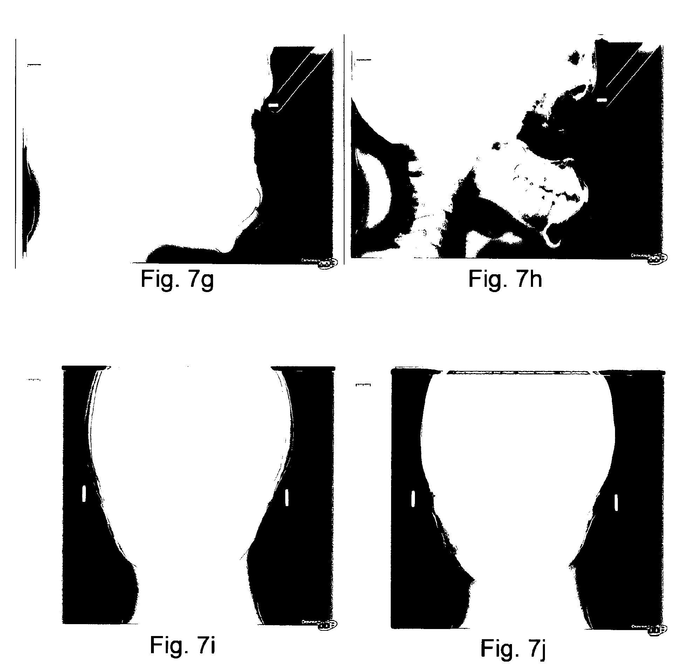

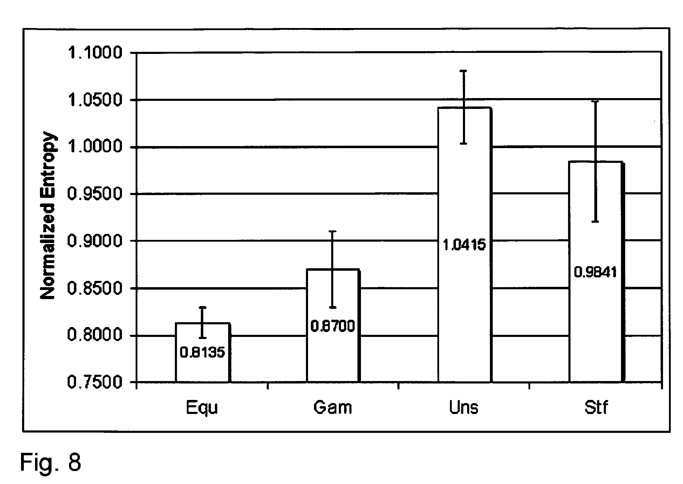

Fig. 8 a diagram showing normalized entropy measures of images processed according to three prior art methods ("Equ", "Gam", "Uns") and according to an embodiment of the present invention ("Stf").

Fig. 1a depicts a typical cephalic radiographic image having a size of 1871 x 2605 pixels.

Fig. 1b shows the image of Fig. 1a after global gamma correction has been performed with a gamma correction parameter of $\gamma = 0,25$. Although the number of gray levels used by the bone pixels (brighter levels) is increased, the dynamic of the darker pixels is compressed so that the visibility of soft tissue pixels has actually been reduced.

Fig. 1c shows the image of Fig. 1a after a histogram equalization step according to the prior art. Both histogram equalization and global gamma correction enhance the pixels that show bone features, but the soft tissue remains dark and tends to mix with the background.

The image shown in Fig. 1d has been obtained from the image of Fig. 1a by means of an Unsharp Masking operation. It is apparent that the high frequencies are enhanced, but noise is increased, whereas bone remains not clearly visible.

For the sake of comparison, the results of processing the image of Fig. 1a with the method of an embodiment of the present invention are shown in Fig. 7h. It is apparent that the image of Fig. 7h represents a considerable enhancement in terms of visibility of both soft tissue and bone features.

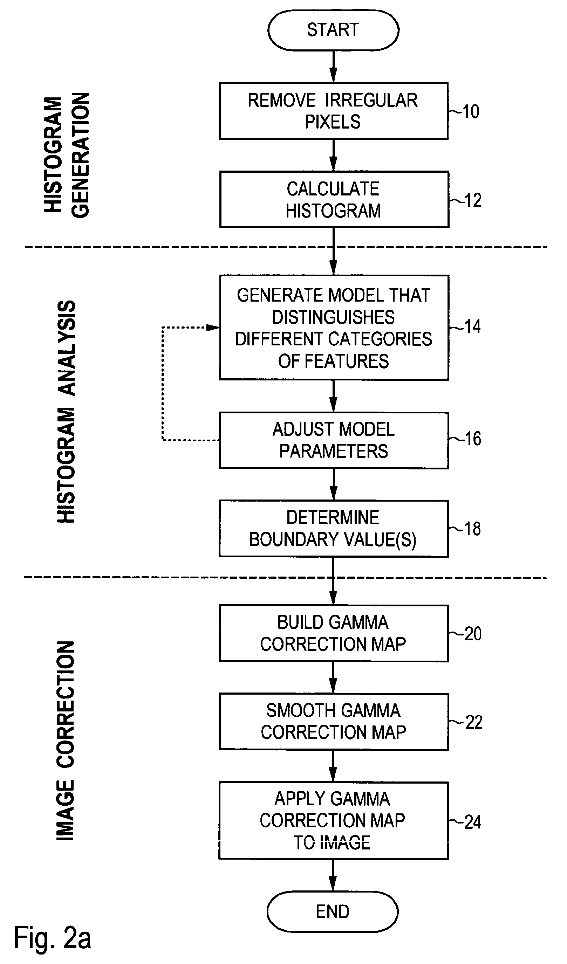

Fig. 2a shows a flow diagram of the method of the present invention in a first sample embodiment. The method can be divided into three parts, each part comprising one or more method steps. It is to be understood that the parts and steps of the method do not necessarily have to be performed in a strictly sequential fashion. In fact, embodiments are contemplated in which the parts and/or steps are performed in an at least partially parallel or an at least partially interleaved fashion.

The three parts of the method shown in Fig. 2a are histogram generation, histogram analysis, and image correction. In histogram generation, a reliable histogram of the image is built. This is done, in step 10 of the present embodiment, by taking out saturated pixels and pixels that belong to borders or logo elements. The histogram is then calculated in step 12 without taking such irregular pixels into account. It is to be understood that, in typical implementations of the present invention, step 10 will be performed partly in conjunction with step 12 and partly as a post-processing step on the histogram obtained in step 12.

The second part of the method, namely histogram analysis, has the object of determining a distinction between different categories of features contained in the image. In the presently described embodiment, three such categories are used, namely background, soft tissue and bony tissue. These categories correspond to three components of the histogram. A model of the histogram is generated in step 14. Here, the histogram is modeled by a mixture (e.g., a weighted linear combination) of one model distribution for each component of the histogram. In the embodiment of Fig. 2a, two Gaussian distributions are used to model the background and soft tissue components of the histogram, respectively, and a Lognormal distribution is used to model the bony tissue component of the histogram.

The parameters of the Gaussian and Lognormal distributions are adjusted in an iterative process (step 16 jumping back to step 14, as shown by the dashed arrow in Fig. 2a) in order to match the model histogram with the actual histogram as accurately as possible. After the iterative process has been completed, a boundary value (e.g., a threshold for the individual pixel values of the image) is determined in step 18. The boundary value serves to distinguish pixels that likely belong to soft tissue features from pixels that likely belong to bony tissue features.

The third part of the method concerns the actual image correction. The boundary value determined in step 18 is used in step 20 to build a gamma correction map that contains a desired gamma correction value for each pixel. This gamma correction map is smoothed in step 22. Finally, the smoothed map is applied to the original image in step 24. Step 24 may be performed by applying a correction formula to each pixel in the image to be processed, the correction formula taking into account the respective gamma correction value for this pixel as defined in the smoothed gamma correction map. In alternative embodiments, a two-dimensional look-up table may be calculated for the image to speed up the image correction process.

The method of Fig. 2a uses a rather sophisticated model in which the modeled histogram is a mixture of several statistical distributions. This makes it necessary to employ an iterative approximation routine for adjusting the model parameters in steps 14 and 16.

Fig. 2b shows an alternative embodiment of the histogram analysis part of the method. Here, a simplified model is used, wherein the modeled histogram consists of segments that are defined by comparatively simple mathematical equations, e.g., equations that define straight line segments or parts of a parabola. The fitting of this model histogram to the actual histogram can then be performed in a simplified and very efficient process, which may or may not require iterative approximation. This process is shown in Fig. 2b as step 26. The boundary value resulting from step 26 is one of the parameters of the model histogram, namely the gray level coordinate (abscissa) of the point where the segment of the histogram that models the soft tissue features goes into the segment of the histogram that models the bone features.

The three parts of the methods outlined above, namely histogram generation, histogram analysis, and image correction, will be explained in more detail in the following sections. Sections 1, 2 and 4 describe the method shown in Fig. 2a, and section 3 covers the variant of the histogram analysis part that has been shown in Fig. 2b. It is to be understood that the details presented below only illustrate certain specific embodiments of the present invention, and should not be construed as limitations of the scope of protection.

### 1. Histogram Generation

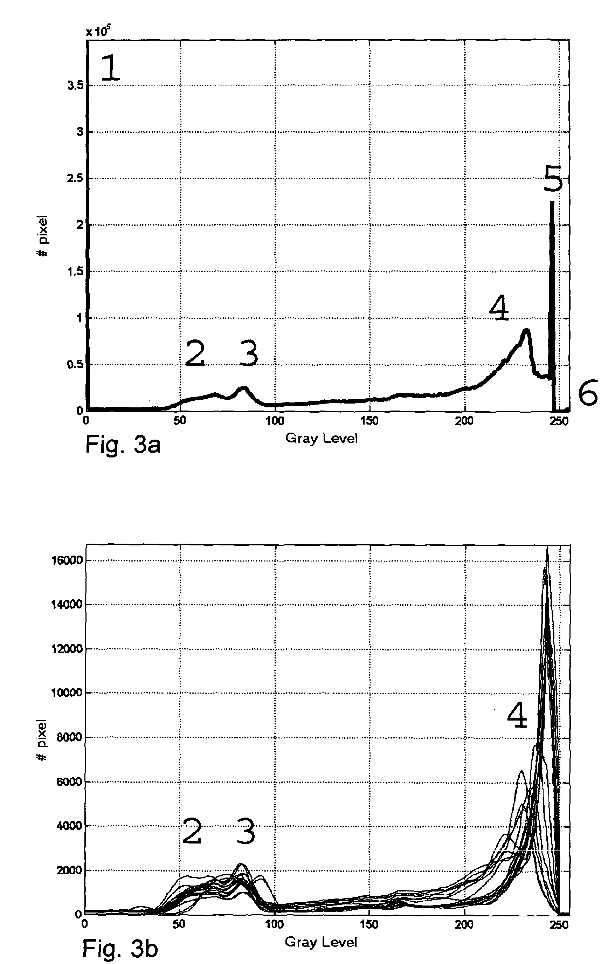

A typical histogram of a cephalic radiographic image is shown in Fig. 3a. This histogram has a consistent shape with six well-defined peaks. Peak 1 is associated with saturated CCD pixels (corresponding to a gray level equal to 0). Peaks 2 and 3 represent the image background; the reason for the presence of a double peak is the use of Automatic Exposure Control (AEC), which has been introduced in the latest generation of radiographic equipment to limit the soft-tissue overexposure in the frontal part of the face of the patient. Peak 4 is associated with the bone structures; it is asymmetric and shows a larger slope for the highest gray levels. Peak 5 corresponds to pixels on the border of the CCD sensor, which receive almost no X-rays. Peak 6 is associated with the digital logo printed on the radiography (corresponding to the maximum gray level, equal to $N_{GL}$ -1, where $N_{GL}$ is the total number of gray level values, e.g., 256 in the present sample embodiments).

The above analysis suggests the following approach for obtaining a reliable histogram. First, pixels on the border of the CCD sensor are discarded. A boundary frame as large as 5 % of the total number of rows and columns is taken out from the image. This is a safe margin to ensure that all pixels that did not fully receive the radiation dose are discarded.

At this point a working histogram of the image, $H_{1F}$, is computed using only the remaining pixels. The peaks associated to saturated pixels (gray level equal to zero) and logo elements (gray level equal to $N_{GL}$ - 1) are now discarded as follows:

$$H_{1F}(0) = H_{1F}(1) \qquad\qquad (1)$$

$$H_{1F}(N_{GL} - 1) = H_{1F}(N_{GL} - 2) \qquad\qquad (2)$$

The resulting histogram $H_{1F}$ is low pass filtered. Fig. 3b shows a diagram in which eighteen low pass filtered histograms have been plotted, one histogram for each of eighteen typical lateral cephalic radiographies. It is apparent that only peaks 2, 3 and 4 are present in $H_{1F}$. The probability that a certain gray level, x, appears in the image, can be computed by normalizing $H_{1F}$. The resulting normalized histogram will be referred to as $H_{1FN}$ in the following.

Soft tissue gray levels are spread between peak 2 and peak 4 (see Fig. 3b). Underexposed and overexposed images generate two different histogram populations: the bone peak is very narrow and high in underexposed radiographies, whereas it tends to decrease and become larger in overexposed radiographies.

### 2. Histogram Analysis (Embodiment Using a Mixture Model)

The purpose of the second method part, i.e., histogram analysis, is to identify a significant threshold ($Th_{Bone}$), which allows separating the brighter bone from the darker soft tissue and background pixels. It is not possible to assign a pre-defined value to $Th_{Bone}$ because the levels of the two families of tissues, and consequently $Th_{Bone}$, vary from image to image, depending on the subject's anatomical characteristics.

The approach that is used in the sample embodiments described in the present section to determine a suitable value of $Th_{Bone}$ is based on mixture models. These form the basis of powerful statistical techniques for density estimation in which the advantages of both parametric and non-parametric methods are combined. Mixture models as such are known. For example, they are described in a general context in the book *Neural Networks for Pattern Recognition* by Christopher M. Bishop, Clarendon Press, Oxford, 1995, pp. 59-73, and in the book *Finite Mixture Models* by Geoffrey McLachlan and David Peel, Wiley, 2000. The disclosure of these books is herewith incorporated into the present document in its entirety.

Mixture models can generally estimate probability densities with complex shapes, such as multimodal histograms, like the one here, using a restricted number of parameters. A mixture distribution is defined as a linear combination of M component densities $p(x|j)$, weighted by the mixing parameters $P(j)$

$$p(x) = \sum_{j=1}^{M} p(x \mid j) \cdot P(j) \qquad (3)$$

with

$$\sum_{j=1}^{M} P(j) = 1 \qquad 0 \le P(j) \le 1 \qquad (4)$$

$$\int p(x \mid j) dx = 1 \qquad (5)$$

In practice, the probability density $p(x)$ is generated as follow: first a component $j$ is chosen with probability $P(j)$; then a data point is generated from the component density $p(x|j)$. Posterior probabilities can be expressed using Bayes' theorem as

$$P(j \mid x) = \frac{p(x \mid j)P(j)}{p(x)} \qquad (6)$$

with

$$\sum_{j=1}^{M} P(j \mid x) = 1 \qquad (7)$$

where $P(j|x)$ is the probability that a particular component $j$ has generated $x$.

In the present sample embodiment, a mixture of two Gaussians and one inverted Lognormal is used to model $H_{1FN}$. Each component of this mixture takes into account the spread of the gray levels associated respectively to background, soft tissue and bone; the characteristic shape of the inverted Lognormal is used to properly describe the asymmetric shape of the bone peak. The inverted Lognormal distribution has different formulations; the one used in the present sample embodiment has a probability density given by

$$p(x) = \frac{1}{\sqrt{2\pi} \cdot \sigma \cdot (N_{GL} - x)} \cdot \exp\left\{ -\frac{[\ln(N_{GL} - x) - \mu]^2}{2\sigma^2} \right\} \qquad (8)$$

and it is defined only for $x < N_{GL}$. Its mean and variance are respectively

$$M = N_{GL} - 1 - \exp\left( \mu + \frac{\sigma^2}{2} \right) \qquad (9)$$

$$S^2 = \exp(\sigma^2 + 2\mu) \cdot (\exp(\sigma^2) - 1) \qquad (10)$$

The other components of the mixture model are Gaussians, described by

$$p(x) = \frac{1}{\sqrt{2\pi}\sigma} \cdot \exp\left(-\frac{(x-\mu)^2}{2\sigma^2}\right) \qquad (11)$$

where $\mu$ and $\sigma^2$ are the mean and the variance of the distribution.

The mixture model is completely defined as soon as the parameters of each distribution $(\mu_j, \sigma_j)$ and the three mixing parameters $P(j)$ have been computed,

For determining these parameters, the likelihood of the parameters for the given dataset is maximized. More easily, the negative log-likelihood, which is given by the value E in the following equation, is minimized:

$$E = -\ln L = -\sum_{n=1}^{N} \ln p(x^n) = -\sum_{n=1}^{N} \ln\{p(x^n \mid j)P(j)\} \qquad (12)$$

where N is the dimension of the dataset.

A closed form solution to compute the parameters by minimizing E in equation (12) is not known. Therefore an iterative method will be adopted. A solution that is known as such is the EM (Expectation Maximization) method. This method is described in detail in the above-referenced books by Bishop and McLachlan/Peel, respectively. The EM method uses equations for updating the parameters of the distributions used in the model histogram in each iteration step. A derivation of such updating equations for standard distributions like Gaussian, Poisson, Lognormal, ... can be found in the above-referenced books.

The inventors have obtained the following updating equations for use with the particular model of the present sample embodiment. The mixing parameters $P(j)$ are updated as follows:

$$P^{new}(j) = \frac{1}{N}\sum_{n=1}^{N} P^{old}(j \mid x) \qquad (X13)$$

The mean and variance of the Gaussian components are updated as follows:

$$\mu_j^{new} = \frac{\sum_{n=1}^{N} P^{old}(j \mid x^n)x^n}{\sum_{n=1}^{N} P^{old}(j \mid x^n)} \qquad (X14)$$

$$\left(\sigma_j^{new}\right)^2 = \frac{\sum_{n=1}^{N} P^{old}(j \mid x^n)(x^n - \mu_j)^2}{\sum_{n=1}^{N} P^{old}(j \mid x^n)} \qquad (X15)$$

For the inverted Lognormal, the updating equations for the parameters, $\mu_j^{new}$ and $\sigma_j^{new}$ are as follows:

$$\mu_j^{new} = \frac{\sum_{n=1}^{N} P^{old}\left(j \mid x^n\right) \ln\left(N_{GL} - x^n\right)}{\sum_{n=1}^{N} P^{old}\left(j \mid x^n\right)} \qquad (X16)$$

$$\left(\sigma_j^{new}\right)^2 = \frac{\sum_{n=1}^{N} P^{old}\left(j \mid x^n\right) \cdot \left[\ln\left(N_{GL} - x^n\right) - \mu_j^{new}\right]^2}{\sum_{n=1}^{N} P^{old}\left(j \mid x^n\right)} \qquad (X17)$$

Equations (X13) - (X17) require that each pixel x is examined, leading to a large computational time. However, the possible values of x are discrete ($N_{GL}$ values), and all the pixels having the same gray value have already been counted in the histogram $H_{1F}$. The updating equations (X13) - (X17) can therefore be simplified. The following equations (13) - (17) are actually used in the present sample embodiment:

$$P(j)^{new} = \frac{1}{N} \sum_{g=0}^{N_{GL}-1} P^{old}\left(j \mid g\right) \cdot H_{1F}(g) \qquad (13)$$

for each j;

$$\mu_j^{new} = \frac{\sum_{g=0}^{N_{GL}-1} P^{old}\left(j \mid g\right) \cdot g \cdot H_{1F}(g)}{\sum_{g=0}^{N_{GL}-1} P^{old}\left(j \mid g\right) \cdot H_{1F}(g)} \qquad (14)$$

$$\left(\sigma_j^{new}\right)^2 = \frac{\sum_{g=0}^{N_{GL}-1} P^{old}\left(j \mid g\right) \cdot \left(g - \mu_j^{new}\right)^2 \cdot H_{1F}(g)}{\sum_{g=0}^{N_{GL}-1} P^{old}\left(j \mid g\right) \cdot H_{1F}(g)} \qquad (15)$$

for the two Gaussians;

$$\mu_j^{new} = \frac{\sum_{g=0}^{N_{GL}-1} P^{old}\left(j \mid g\right) \cdot \ln\left(N_{GL} - g\right) \cdot H_{1F}(g)}{\sum_{g=0}^{N_{GL}-1} P^{old}\left(j \mid g\right) \cdot H_{1F}(g)} \qquad (16)$$

$$\left(\sigma_j^{new}\right)^2 = \frac{\sum_{g=0}^{N_{GL}-1} P^{old}\left(j \mid g\right) \cdot \left[\ln\left(N_{GL} - g\right) - \mu_j^{new}\right]^2 \cdot H_{1F}(g)}{\sum_{g=0}^{N_{GL}-1} P^{old}\left(j \mid g\right) \cdot H_{1F}(g)} \qquad (17)$$

for the inverted Lognormal.

It should be noted that the term $\sum_{g=0}^{N_{GL}-1} P^{old}(j \mid g) \cdot H_{1F}(g)$ is common to the updating equations for the mixing parameters (equation 13) and for the parameters for the components (equations 14-15 and 16-17, respectively). Therefore it is sufficient that this term is computed only once for all the components.

To get a reliable estimate and maximize the speed of convergence, the parameters are initialized to a mean value that has been pre-computed on the basis of a set of typical test images. The above updating operations are now applied a sufficient number of times. After the mixture model parameters have converged, the 1st Gaussian component of the histogram model corresponds to the background; the 2nd Gaussian component is associated with the soft tissue; and the inverted Lognormal 3rd component describes the bone's typical gray levels.

The threshold that separates the soft tissue from the bone structures, $Th_{Bone}$, is now set so that the following function is minimized:

$$\int_0^{Th_{Bone}} p(x \mid 3)P(3)dx + \int_{Th_{Bone}}^{N_{GL}} p(x \mid 2)P(2)dx \qquad (18)$$

that is, the probability to assign x to the wrong component $j$ is minimized, for $j = 2$ or $j=3$.

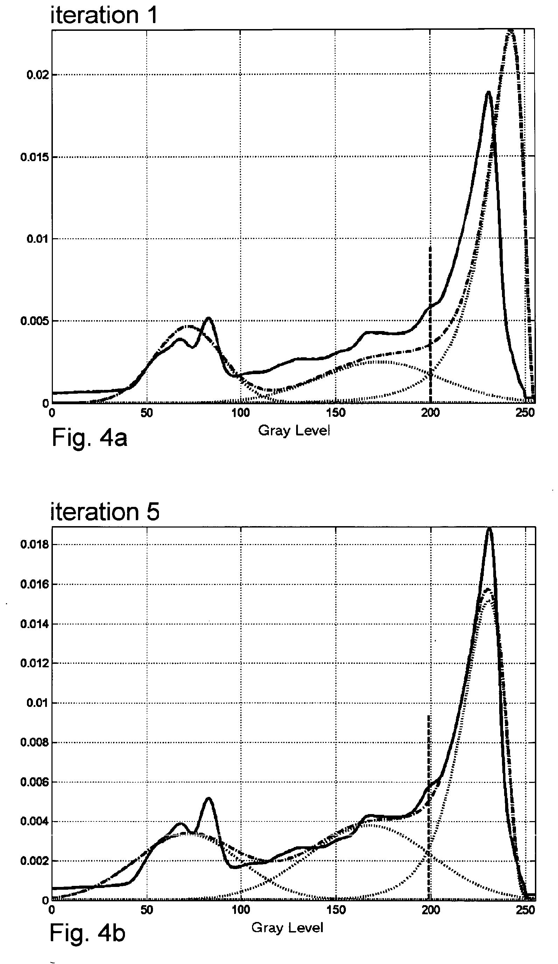

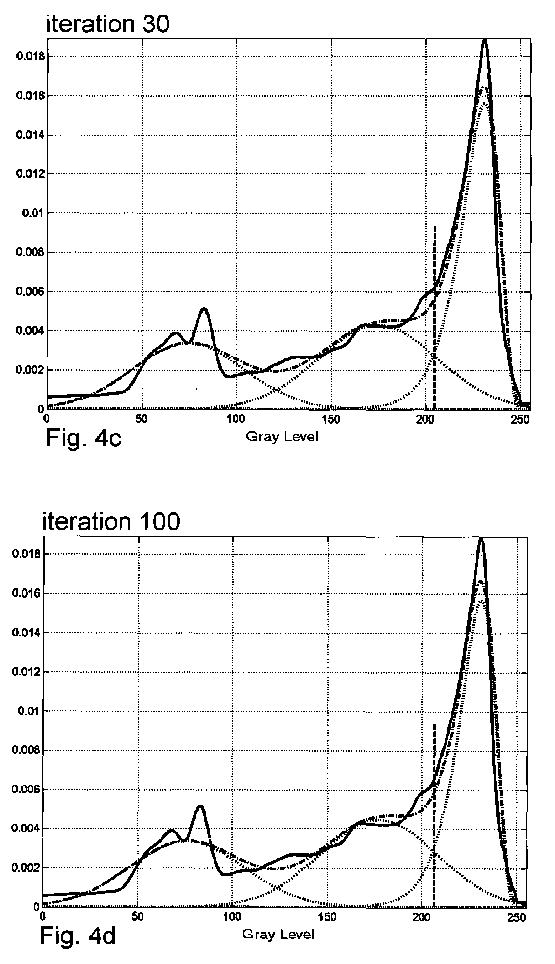

The diagrams shown in Fig. 4a - Fig. 4h illustrate the process described above. In each of Fig. 4a - Fig. 4f, the normalized histogram of the original image, $H_{1FN}$, is plotted with a continuous line (—). The probability density of each gray level, computed by the mixture model, is plotted with a dash-dot line (-·-·-·-·), and the probability densities of the three components are plotted with dotted lines (·····). The computed boundary value $Th_{Bone}$ is shown in each diagram as a vertical dashed line. The histogram curves and the boundary value $Th_{Bone}$ are shown at iteration step 1 (Fig. 4a), iteration step 5 (Fig. 4b), iteration step 30 (Fig. 4c), and iteration step 100 (Fig. 4d) of the EM algorithm.

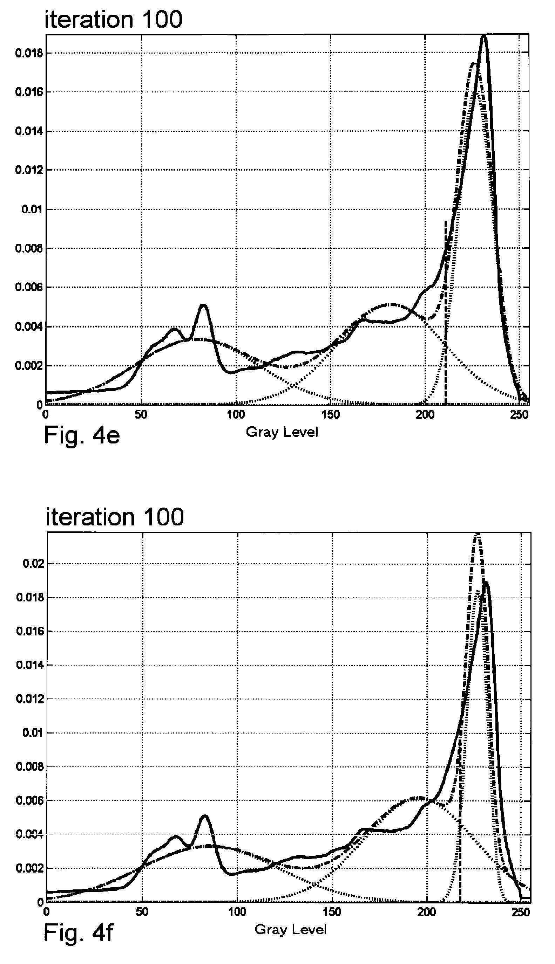

Fig. 4e depicts the fitting of the histogram through a mixture of three Gaussians. Fig. 4f shows the fitting through a mixture of two Gaussians and one Inverted Poisson.

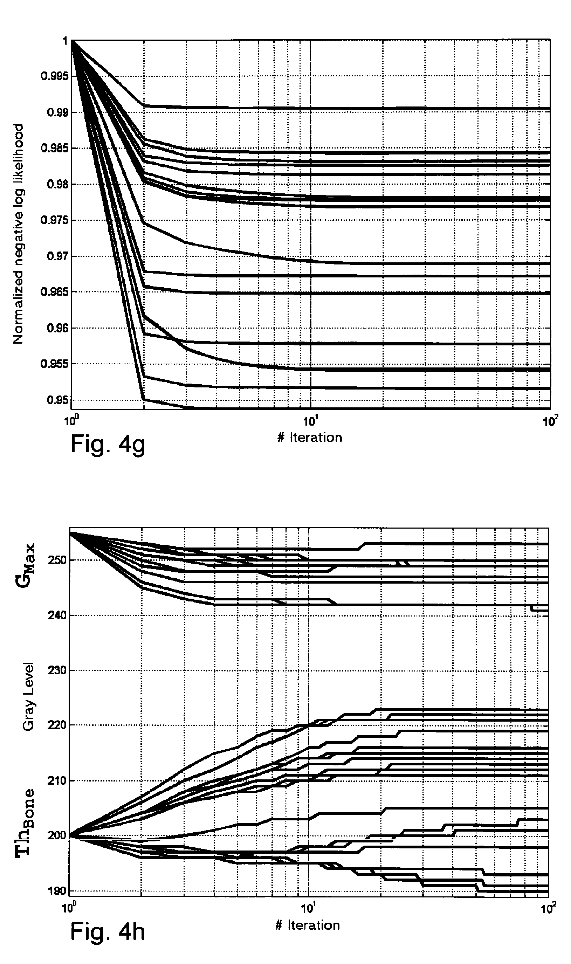

Fig. 4g illustrates the negative log-likelihood E according to equation (12), which has been normalized to its initial value, versus the number of iteration steps for eighteen typical radiographies. The respective values of $Th_{Bone}$ and $G_{Max}$ for these eighteen radiographies are plotted in Fig. 4h.

### 3. Histogram Analysis (Embodiment Using a Segment Model)

The embodiment described above used a rather complex model with a mixture of statistical distributions for the three categories background, soft tissue and bone. The complexity of this model may be a problem in some circumstances. In the present section, a simplified model will be described that only distinguishes two components of the histogram. The feature categories corresponding to these two components are soft tissue (including background) on the one hand and bone on the other hand. More importantly, the model histogram of the embodiment described in the present section is not a mixture of statistical distributions, but is formed by segments of simple functions. This allows calculation of the boundary value $Th_{Bone}$ in a simplified process.

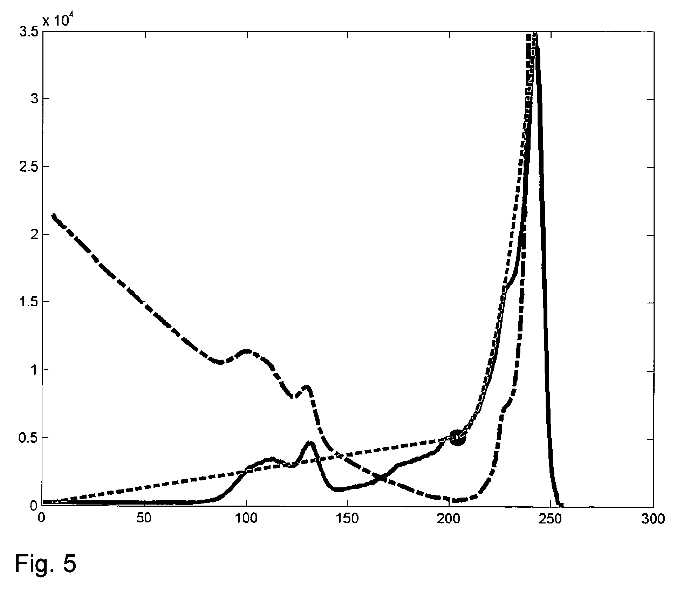

Fig. 5 shows the working histogram $H_{1F}(x)$ plotted as a continuous line (—). The simplified histogram model is shown as a dashed line with uniform short dashes (-----). In the embodiment shown in Fig. 5, the histogram model consists of a left-hand segment, which is just a line segment, and a right-hand segment, which is a piece of a parabola. Both segments join at a joining point, which is shown as a spot in Fig. 5. The x coordinate (abscissa) of the joining point represents the boundary value $Th_{Bone}$ that is to be estimated; this value will be called $G_{Threshold}$ in the following.

More formally, let x be the gray level and $y$ the histogram value. The simplified model according to the present sample embodiment is composed of the following two segments:

a) a line segment, $y_L(x) = L_1 x + L_0$, whose parameters are determined so that the line segment passes through $[G_{Min}, H_{1F}(G_{Min})]$ and $[G_{Threshold}, H_{1F}(G_{Threshold})]$; and

b) a piece of a parabola, $y_p(x) = P_2 x^2 + P_1 x + P_0$, whose parameters are determined so that the parabola passes trough $[G_{Threshold}, H_{1F}(G_{Threshold})]$ and $[G_{Max}, H_{1F}(G_{Max})]$.

The following designations have been used in the above equations:

- $L_0$ and $L_1$ are the line parameters,

- $P_0$, $P_1$ and $P_2$ are the parabola parameters,

- $H_{1F}(x)$ is the working histogram value, computed for gray level x , as described above in section 1,

- $G_{Min}$ is the minimum significant gray level in the image; it is calculated as the lowest gray level x for which $H_{1F}(x + 1) > H_{1F}(x)$ holds, i.e., from which the histogram starts increasing,

- $G_{Max}$ is the gray level corresponding to the maximum value of $H_{1F}(x)$, computed in the right-hand segment of the histogram where $x > (2^N-1)/2$, and

- $G_{Threshold}$ is the boundary value to be estimated, which separates bone from soft tissue.

The parameters $[L_i, P_i]$ of the simplified model are estimated such that the slope of the segment is equal to the slope of the parabola (first derivative) in $G_{Threshold}$, that is:

$$L_1 = 2P_2 G_{Threshold} + P_1 \qquad (S1)$$

To estimate the five parameters of the model, $[L_i, P_i]$, five equations are required. These are equation (S1) and the following four conditions relating to extreme points:

- the straight line segment runs through $[G_{Min}, H_{1F}(G_{Min})]$,

- the straight line segment runs through $[G_{Threshold}, H_{1F}(G_{Threshold})]$,

- the parabola runs through $[G_{Threshold}, H_{1F}(G_{Threshold})]$, and

- the parabola runs through $[G_{Max}, H_1 F(G_{Max})]$.

Thus the five parameters of the model are completely defined because a sufficient number of equations is provided. The four conditions given above constrain the extremes of the line segment and the piece of the parabola. Equation (S1) is an additional condition, which allows closing the system.

The threshold gray level, $G_{Threshold}$, is then computed by minimizing the following error, E , corresponding to the quadratic distance between the simplified model and the working histogram $H_{1F}(x)$ :

$$E(x) = \sum_{t=GMin}^{x} (y_L(t) - H_{1F}(t))^2 + \sum_{t=x}^{GMax} (y_P(t) - H_{1F}(t))^2 \qquad (S2)$$

To solve the system, the value of E is computed for all the gray levels (x values) between $G_{min}$ and $G_{max}$. Then, $G_{threshold}$ is determined as the value of x for which the corresponding E(x) is minimum.

The dashed line with alternating short and long dashes (—·—·—) in Fig. 5 shows the value of E(x) over the possible range of x values. It is apparent that the error E(x) reaches its minimum for $x \approx 205$, and consequently $G_{threshold}$ will be set to 205 in this particular run of the image enhancement method. This value will be used as the boundary value $Th_{Bone}$ in the next part of the method, namely the image correction.

## 4. Image Correction

After a boundary value $Th_{Bone}$ has been determined according to one of the methods described above, the radiographic image is corrected in order to increase the visibility of its bone and soft tissue features.

In a very simple sample embodiment, pixel-to-pixel gamma correction is applied according to the following formula (19), where I(i,j) is the gray level of the pixel (i,j) in the original image, I'(i,j) is the gray level of the pixel (i,j) in the corrected image, and $\gamma(i,j)$ is the gamma value as given in a correction map defined by the following formula (20) for two pre-set gamma value constants, namely $\gamma_{Soft\_tissue}$ and $\gamma_{Bone}$:

$$I'(i, j) = (N_{GL} - 1)\left[\frac{I(i,j)}{N_{GL} - 1}\right]^{\frac{1}{\gamma(i,j)}} \qquad (19)$$

where

$$\lambda(i, j) = \begin{cases} \gamma_{Soft\_tissue} & \text{if} \quad I(i, j) \leq Th_{Bone} \\ \gamma_{Bone} & \text{if} \quad I(i, j) > Th_{Bone} \end{cases} \qquad (20)$$

In effect, each pixel (i,j) such that I(i,j) ≤ $Th_{Bone}$ is modified by using $\gamma(i,j) = \gamma_{Soft\_tissue}$, whereas each pixel (i,j) such that I(i,j) > $Th_{Bone}$ is modified by using $\gamma(i,j) = \gamma_{Bone}$.

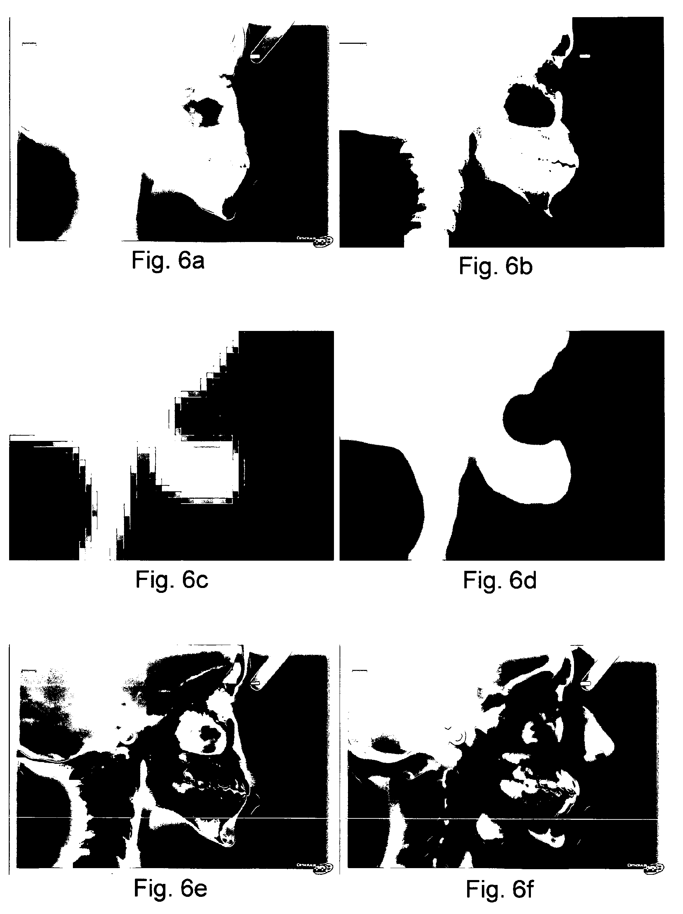

Although the above simple procedure is fast, it does not take into account that the transition between bone and soft tissue is usually not as sharp as a pixel transition. Consequently, in preferred embodiments of the invention, the γ values are smoothed in the spatial domain to avoid strong artifacts like those shown in Fig. 6e. An example of an embodiment that includes a step of smoothing the gamma correction map is shown in Fig. 2a and described in the following. In this embodiment, a binarized gamma correction map, $\Gamma_b(.)$, is created. The gamma correction map $\Gamma_b(.)$ contains either the value $\gamma_{Soft\_tissue}$ or the value $\gamma_{Bone}$. Fig. 6b shows an example of a binarized gamma correction map $\Gamma_b(.)$, which has been obtained from the cephalic radiographic image shown in Fig. 6a.

After the binarized gamma correction map $\Gamma_b(.)$ has been obtained, it is smoothed by a spatial filtering process to obtain the final gamma map, $\Gamma_f(.)$, which will be applied to the image. The spatial filtering process starts with the step of down-sampling $\Gamma_b(.)$ into a downsampled map $\Gamma_d(.)$. For this purpose, $\Gamma_b(.)$ is subdivided into square blocks, $B_{l,m}$, of size TP x TP, where TP is a pre-set parameter of the image correction method.

For each block $B_{l,m}$ the downsampled map $\Gamma_d(l,m)$ contains the mean gamma value inside the block $B_{l,m}$. The downsampled map $\Gamma_d(.)$ is then spatially filtered by using a 3x3 moving average; the result is shown in Fig. 6c. This procedure is equivalent to an undersampling of $\Gamma_b(.)$ using partially overlapping windows. The final gamma map $\Gamma_f(.)$ is then obtained by upsampling $\Gamma_d(.)$ through a bilinear interpolation scheme; Fig. 6d shows an example of the results, i.e., the final gamma map $\Gamma_f(.)$ that will be used for image correction.

A further step is performed in the present sample embodiment to take advantage of the full dynamics of the gray levels. This step is that a linear stretching with saturation is carried out on the histogram $H_{1F}$ before performing local gamma correction. The highest significant gray level of the image, $G_{Max}$, can be identified as

$$G_{Max} = \min(N_{GL} - 1, M + S) \qquad (21)$$

where M and S are respectively the mean and the standard deviation of the inverted Lognormal of the mixture model.

Combination of the linear stretching according to equation (21) with saturation and local gamma correction according to equations (19) and (20) gives the following final correction formula for each pixel (i,j):

$$I'(i, j) = \begin{cases} (N_{GL} - 1) \cdot \left[\dfrac{I(i,j)}{G_{Max}}\right]^{\frac{1}{\Gamma_f(i,j)}}, & I(x, y) < G_{Max} \\ (N_{GL} - 1), & \text{elsewhere} \end{cases} \qquad (22)$$

The result obtained by applying equation (22), which uses the final gamma map $\Gamma_f(.)$, to the original image of Fig. 6a is shown in Fig. 6f. Here, the parameters $\gamma_{Bone} = 0,25$, $\gamma_{Soft\_Tissue} = 2,2$ and TP = $N_{ROW}$ / 24 have been used.

In some embodiments, equation (22) is directly applied to each pixel. However, in other embodiments, a look-up table (LUT) is used for the image correction. Implementing equation (22) through the LUT provides for particularly fast processing times, i.e., interactive image generation rates.

In order to generate the LUT, the final gamma map $\Gamma_f(i,j)$ is discretized into $N_v$ values, $\Gamma_0, ..., F_{NV-1}$. For each gray level, $I_p$, 0 ≤ p ≤ $N_{GL}$-1, and for each gamma value, $\Gamma_k$, 0 ≤ k ≤ $N_v$-1, the corrected gray level, I'p, is computed through equation (22) and stored in the LUT. The LUT therefore is two-dimensional with a total of $N_{GL}$ x $N_v$ entries. After the LUT has been obtained, each pixel I(i,j) is corrected by accessing the LUT table in position I(i,j), $\Gamma$(i,j) according to the following formula:

$$I'(i,j) = LUT[I(i,j), \Gamma(i,j)] \tag{23}$$

### 6. Experimental Results

The method shown in Fig. 2a and described above in sections 1, 2 and 4, with an implementation of the image correction part using a LUT, was implemented in C++, on an Athlon 2400+ processor running at 1,79 GHz, 256M RAM. The method was clinically tested on a wide variety of cephalic, lateral radiographies of size 1871 x 2606 pixels and cephalic, frontal radiographies, of size 2304 x 2607, on 8 bits (i.e., 256 gray levels), acquired using the Gendex Orthoralix 9200 DDE®. In the experiments, the image enhancement method of the present invention was consistently able to produce clear visibility of both bone and soft tissue with a wide range of exposures, including underexposed and overexposed radiographies. Quantitative results will be reported in the following for a test set of eighteen typical images. Some results are shown Fig. 7a - Fig. 7j.

Fig. 7a and Fig. 7c represent two examples of original lateral cephalic radiographies. The results of processing these images as described above with the default settings of $\gamma_{Bone}$ = 0,25, $\gamma_{Soft\_Tissue}$ = 1,5 and TP = $N_{ROW}$ / 36 are shown in Fig. 7b and Fig. 7d, respectively.

Fig. 7e and Fig. 7f show the original and processed versions, respectively, of an overexposed lateral cephalic radiography. Here, the parameters $\gamma_{Bone}$ = 0,2, $\gamma_{Soft\_Tissue}$ = 2 and TP = $N_{ROW}$ / 24 have been used. In the original image (Fig. 7e), the contrast between bone and soft tissue is quite high, but the soft tissue tends to merge with the background. Processing the image according to the method described above had the effect that the bone structures became more visible, while chin and soft tissue were clearly distinguishable from the background (Fig. 7f).

Similar results were obtained for underexposed radiographies. Fig. 7g shows an example of an underexposed lateral cephalic radiography. Fig. 7h represents the effects of processing the image of Fig. 7g with the method described above, using the processing parameters $\gamma_{Bone}$ = 0,15, $\gamma_{Soft\_Tissue}$ = 1 and TP = $N_{ROW}$ / 36. Here the soft tissue is visible, but the bone dynamic is very compressed; moreover, the contrast between soft tissue and bone is very low. The use of a low $\gamma_{Bone}$ parameter (e.g., $\gamma_{Bone}$ = 0,15), as shown in Fig. 7h, allows increasing the bone structure visibility, whereas it leaves the soft tissue almost untouched.

Fig. 7i and Fig. 7j demonstrate the effect of the method described above for a frontal cephalic radiography. The original image is shown in Fig. 7i, and Fig. 7j represents the processed version with $\gamma_{Bone}$ = 0,2, $\gamma_{Soft\_Tissue}$ = 1 and TP = $N_{ROW}$ / 36.

Total processing time for the method was about one second for each image, on average. In particular, for images with a size of 1871 x 2606 pixels, processing time, Tp, was 1,08 $\pm$ 0,01 s (mean $\pm$ 2 standard deviations): only 6% of Tp is devoted to histogram computation and its analysis through the mixture model. Construction of $\Gamma_b$, its smoothing to obtain $\Gamma_f$, and gray level correction using the LUT, take 17%, 42% and 17% of Tp, respectively. The remaining 18% of $T_P$ is required by memory allocation. These times allow working at interactive rates and allow adjusting the parameters ($\gamma_{Bone}$, $\gamma_{Soft\_Tissue}$ and TP) to obtain the subjectively optimal result.

Besides the above qualitative evaluation, quantitative assessment of the method of the present invention has been carried out through an analysis of Shannon entropy. Shannon entropy has been chosen as an index for quantitative assessment since it quantifies the information contained in an image, taking into account only the distribution of the gray levels. Shannon entropy is defined by the following formula:

$$H = -\sum_{x=0}^{N_{GL}-1} p(x)\log_2(p(x)) \tag{24}$$

where $p$(x) is the probability of the gray level x in the image. In order to compare the processing effect on a population of images with spread H values, normalized entropy, $H_N$, has been adopted. This is defined as the ratio of the entropy of the treated image with respect to that of the original image.

The $H_N$ values (mean normalized entropy $\pm$ 2 standard deviations) for a set of eighteen typical radiographies are shown in Fig. 8. The images have been processed by equalization ("Equ"), global gamma correction ("Gam" - $\gamma$ = 0,25), unsharp masking ("Uns" - mask dimension = 25x25, gain = 2), and the method of the present invention ("Stf" - $\gamma_{Soft\_tissue}$ = 1,5, $\gamma_{Bone}$ = 0,25, TP = 52). It is apparent that histogram equalization ($H_N$ = 0,814 $\pm$ 0,017) and global gamma correction ($H_N$ = 0,870 $\pm$ 0,041) produce a significant decrease in the information content of the images, while unsharp masking increases it by a small but statistically significant quantity ($H_N$ = 1,041 $\pm$ 0,038). On the other hand, the method of the present invention ($H_N$ = 0,991 $\pm$ 0,039) does not significantly alter the information contained in the image.

The results of the quantitative analysis shown in Fig. 8 are congruent with visual inspection. Both equalization and global gamma correction decrease the amount of information in the image, and visual inspection indeed reveals that soft tissue gray levels are compressed and become less visible after the transformation. Unsharp masking, on the contrary, being fundamentally a derivative technique, introduces noise and edge artifacts in the treated radiograms, leading to a little increase in the information content. The method of the present invention does not significantly alter the information content of the image: it increases the readability by application of an adaptive monotonic transformation, whose parameters depend on the local gray levels of the image.

### 7. Further Remarks and Alternative Embodiments

The components of the mixture model as described above in section 2 have been carefully chosen, and the inventors presently believe that this particular mixture model is the best way of practicing the invention. However, the invention is generally not limited to one particular mixture model, and alternative embodiments are envisaged in which the model has different or additional or fewer components.

For example, the two background distributions (peaks 2 and 3 in Fig. 3a) have been represented by a single Gaussian distribution in the model of section 2, but alternative embodiments are envisaged in which the background is modeled with two separate components, e.g., two Gaussian distributions.

In the mixture model as described in section 2, soft tissue was represented with a Gaussian distribution of gray levels with wide amplitude. This choice presented no particular problems. On the other hand, selection of a model for the bone tissue was not so straightforward. This is because the histogram associated with bone tissue is rather complex, having a marked asymmetric shape and exhibiting peaks of different positions and amplitudes, which depend on the exposure parameters: underexposure increases the peak amplitude and peak position and decreases the peak width.

Three types of mixtures, in particular, have been tested: three Gaussians, two Gaussians plus one inverted Poisson and two Gaussians plus one inverted Lognormal. With the first two mixtures, it was found difficult to fit the bone peak, as shown in Fig. 4e and Fig. 4f. In the model of Fig. 4e, the third Gaussian component makes it difficult to capture the asymmetry of the bone peak in $H_{1FN}$. In the model of Fig. 4f, the asymmetry of the Poisson distribution is also not sufficient to properly describe the asymmetry of the bone peak. As a consequence, the threshold $Th_{Bone}$ tends to be set too high.

The inverted Lognormal distribution, as used in the model described above in section 2, has proved to work properly, both for underexposed radiographies (which have a narrow bone peak) and for overexposed images (which have a more widely spread histogram). Overall, the three components of the model of section 2 provide an excellent representation of background, soft and bony tissue, as shown in Fig. 4d. This mixture is validated also by the quantitative comparison of the respective likelihood values obtained from the three mixtures. If the negative log likelihood value of the mixture composed of two Gaussians and one inverted Lognormal is normalized to 1, then the corresponding value for the mixture of three Gaussians is 1,0120 $\pm$ 0,0105 (mean $\pm$ 2 standard deviations), and the value for the mixture of two Gaussians and one inverted Poisson is 1,0136 $\pm$ 0,0124 for the eighteen test images.

The analytical shape of the inverted Lognormal distribution according to equation (8) is particularly advantageous because of the possibility to derive closed analytical equations to update the parameters. This form is suitable for use with the EM method, which produces a fast and stable convergence: less than 60 ms are required to perform 100 iterations.

In the set of eighteen typical test images, no change in the average gray levels associated with $Th_{Bone}$ and $G_{Max}$, respectively, was observed after 100 iterations (Fig. 4h). The negative log-likelihood according to equation (12) did not decrease significantly already after 50 iterations, as shown in Fig. 4g.

Experiments have further shown that initialization is not critical in the mixture model of section 2: positioning the three components equally spaced in the gray level domain, and setting the variance of the three components to $N_{GL}/10$, increased the computational time by less than 6 ms (the number of iterations to achieve the same figures increases by less than 10 %).

The transformation according to equation (22) has been found to be especially advantageous because of its combination of linear stretching, saturation and local gamma correction. Due to the saturation component, all gray levels higher than $G_{Max}$ are clipped to $N_{GL}-1$ in the filtered image. As $G_{Max}$ is properly estimated by the mixture, only almost empty gray levels are clipped. On the other hand, these levels are recovered by stretching, which leads to an increase in image contrast.

Three parameters, namely $\gamma_{Soft\_Tissue}$, $\gamma_{Bone}$ and TP, are used in the method described above. The default values of these parameters are: $Y_{Bone}$ = 0,25, $\gamma_{Soft\_Tissue}$ = 1,5 and TP = $N_{ROW}$ / 36. This default setting yields good results over a wide variety of images, as demonstrated in Fig. 7a - Fig. 7d. However, thanks to the computational efficiency of the proposed method, the user can modify the values of these parameters in an interactive way to obtain the best subjective image quality. Even highly underexposed or highly overexposed images can be recovered by a suitable setting of the method parameters. Underexposure can generally be corrected by using a small value for $\gamma_{Bone}$ (for instance, $\gamma_{Bone}$ = 0,15 as in Fig. 7h), while a high value of $\gamma_{Soft\_Tissue}$ allows recovery of overexposed soft tissue (for instance, $\gamma_{Soft\_Tissue}$ = 2 in Fig. 7f).

According to one of the sample embodiments described in section 4, the gamma map is smoothed in the spatial domain to avoid strong artifacts (Fig. 6e). The proposed four step procedure leads to a bilinear interpolation scheme to generate $\Gamma_f$. It is envisaged to use a spline-based upsampling in alternative embodiments, but then additional measures need to be taken to avoid oscillations in $\Gamma_f$. In further alternative embodiments, smoothing of the binary map $\Gamma_b$ is performed by a simple moving average filter, which can be efficiently implemented in the spatial domain to work in real time. This simple solution, however, tends to generate artifacts in $\Gamma_f$ due to the square shape of the moving window. More refined techniques, in which the filter scale is selected locally on the basis of scale-space analysis, are also envisaged and may be used in future developments where more computational power is available.

### 8. Summary

The *Soft Tissue Filtering* method described in the present document corresponds to a local monotonic non-linear stretching of the gray scale, where soft tissue and bone gray level ranges are enlarged to make the structures more visible. As a result, the histogram of bone and soft tissue partially overlaps. This is not a problem for the primary fields of use envisaged for the present invention, where a clinician needs to perform precise identification of anatomical features, alterations in the anatomy of a patient, and visualization of post-operative aesthetic modifications.

The core of the embodiment described in sections 1, 2 and 4 is an innovative modeling of the histogram through an adequate mixture model, which allows a reliable clustering of the cephalic images in a very short time (less than 60 ms).

The image enhancement method of the present invention constitutes a powerful tool for clearly visualizing both soft tissue and bone in the same image. Moreover, the image enhancement method can be integrated in tools for automatic cephalometric orthodontia. The speed of operation and the intuitive modification of the free parameters are important benefits. The approach described in the present document can be adapted to all types of medical images that have a well defined multi-modal histogram, and the approach can be used in all medical fields where features of different tissues in a single image need to be clearly visualized.

The details mentioned above are not to be construed as restrictions of the scope of the present invention, but rather as examples of possible embodiments. Many further alternatives are possible and will be apparent to the person skilled in the art. Accordingly, the scope of the present invention is not be determined from the above examples, but rather from the appended claims and their legal equivalents.

### Claims

1. A method for enhancing the visibility of features in a radiographic image, the features belonging to at least a first and a second category of features, the image being composed of a plurality of image elements, each image element of the plurality of image elements having at least one respective value, the method comprising the steps of:

   - determining a histogram of the image showing the distribution of the values of the image elements in the image,
   - analyzing the histogram in order to determine a distinction between values of image elements that more likely show a feature of the first category and values of image elements that more likely show a feature of the second category,
   - applying a correction to at least some of the image elements, wherein an image element that, according to the determined distinction, more likely shows a feature of the first category is corrected differently than an image element that, according to the determined distinction, more likely shows a feature of the second category.

2. The method of claim 1, wherein the first category of features comprises features of soft tissue shown in the image, and the second category of features comprises features of bone shown in the image.

3. The method of claim 1 or claim 2, wherein a third category of features comprises features of the background shown in the image.

4. The method of one of claims 1 to 3, wherein irregular image elements are disregarded in the step of determining the histogram.

5. The method of claim 4, wherein the irregular image elements comprise at least one of the following:

- image elements near the border of the image,
- image elements that represent or contain saturated pixels, and
- image elements that contain electronically inserted information.

6. The method of one of claims 1 to 5, wherein the step of analyzing the histogram comprises the step of fitting a model histogram to the actual histogram.

7. The method of claim 6, wherein the model histogram comprises a plurality of components.

8. The method of claim 7, wherein each component of the plurality of components corresponds to at least one of the categories of features shown in the image.

9. The method of claim 7 or claim 8, wherein the model histogram is a mixture, in particular a weighted linear combination, of the plurality of components.

10. The method of claim 9, wherein each component of the plurality of components is a statistical distribution, in particular a Gaussian and/or Poisson and/or Lognormal and/or inverted Gaussian and/or inverted Poisson and/or inverted Lognormal distribution.

11. The method of claim 7 or claim 8, wherein each component of the plurality of components forms a segment of the model histogram.

12. The method of claim 11, wherein each component of the plurality of components is defined by a linear or quadratic or cubic equation.

13. The method of one of claims 6 to 12, wherein the step of fitting the model histogram to the actual histogram maximizes the likelihood of the observed image data.

14. The method of one of claims 6 to 13, wherein the step of fitting the model histogram to the actual histogram comprises an iterative approximation process.

15. The method of one of claims 1 to 14, wherein the distinction comprises at least one boundary value that distinguishes image elements that more likely show a feature of the first category from image elements that more likely show a feature of the second category.

16. The method of one of claims 1 to 15, wherein the correction applied to each image element comprises a gamma correction with a respective gamma correction value, the gamma correction value for each image element being determined or at least influenced by the determined distinction between the values of the image elements.

17. The method of claim 16, wherein the gamma correction values applied to each image element are defined by a gamma correction map that has been smoothed in the spatial domain.

18. The method of claim 16 or claim 17, wherein a two-dimensional look-up table is used in the process of applying the correction to the image elements, the look-up table associating pairs of gamma correction values and original image element values to corrected image element values.

19. The method of one of claims 1 to 18, wherein the correction further includes a linear stretching of the range of values of the image elements and/or a correction of the saturation of the image elements.

20. The method of one of claims 1 to 19, wherein each image element is an individual pixel, and wherein the value of each image element is a gray level of this individual pixel.

21. The method of one of claims 1 to 20, wherein the radiographic image is a cephalic image.

22. An apparatus, in particular a digital X-ray apparatus or an image processing apparatus, that is adapted for performing the method of one of steps 1 to 21.

23. A computer-readable data carrier comprising a plurality of program instructions for causing a processor to perform the method of one of steps 1 to 21.

Fig. 1a  (PRIOR ART)

Fig. 1b  (PRIOR ART)

Fig. 1c  (PRIOR ART)

Fig. 1d  (PRIOR ART)

HISTOGRAM ANALYSIS

CALCULATE
PARAMETERS
OF SIMPLE MODEL,
INCLUDING
BOUNDARY VALUE(S) ~26

Fig. 2b

Fig. 2a

Fig. 3a

Fig. 3b

iteration 1

Fig. 4a

iteration 5

Fig. 4b

## iteration 30

Fig. 4c

## iteration 100

Fig. 4d

iteration 100

Fig. 4e                    Gray Level

iteration 100

Fig. 4f                    Gray Level

Fig. 4g

Fig. 4h

Fig. 5

Fig. 6a

Fig. 6b

Fig. 6c

Fig. 6d

Fig. 6e

Fig. 6f

Fig. 7a

Fig. 7b

Fig. 7c

Fig. 7d

Fig. 7e

Fig. 7f

Fig. 7g

Fig. 7h

Fig. 7i

Fig. 7j

Fig. 8